(19) Europäisches Patentamt

European Patent Office

Office européen des brevets

(11) **EP 0 923 927 B1**

(12) **FASCICULE DE BREVET EUROPEEN**

(45) Date de publication et mention de la délivrance du brevet:
**30.05.2001 Bulletin 2001/22**

(51) Int Cl.$^7$: **A61K 7/02**

(21) Numéro de dépôt: **98402650.0**

(22) Date de dépôt: **23.10.1998**

(54) **Composition cosmétique pulvérulente longue tenue comprenant une dispersion de particules de polymère dans une phase grasse liquide**

Pulverkosmetikzusammensetzung für langen Gebrauch enthaltend eine Kunststoffdispersion in einer flüssigen Ölphase

Long-wearing powderous cosmetic composition comprising a dispersion of polymer particles in a liquid oil phase

(84) Etats contractants désignés:
**DE ES FR GB IT**

(30) Priorité: **22.12.1997 FR 9716253**

(43) Date de publication de la demande:
**23.06.1999 Bulletin 1999/25**

(73) Titulaire: **L'OREAL**
**75008 Paris (FR)**

(72) Inventeurs:
- **Poterie, Valérie de la**
  **77820 Le Chatelet en Brie (FR)**
- **Mougin, Nathalie**
  **75011 Paris (FR)**
- **Lemann, Patricia**
  **94000 Creteil (FR)**

(74) Mandataire: **Lhoste, Catherine**
**L'OREAL-DPI**
**6 rue Bertrand Sincholle**
**92585 Clichy Cédex (FR)**

(56) Documents cités:
**EP-A- 0 545 786**      **EP-A- 0 566 442**
**FR-A- 2 679 444**

# Description

La présente invention a trait à une composition pulvérulente contenant un polymère dispersible dans une phase grasse destinée en particulier aux domaines cosmétique, dermatologique, pharmaceutique et hygiénique. Plus spécialement, l'invention se rapporte à une composition pulvérulente de longue tenue et sans transfert pour le soin et/ou le maquillage de la peau aussi bien du visage que du corps humain, ainsi que des lèvres.

Cette composition peut se présenter notamment sous forme de poudre libre, pressée ou compactée pour le maquillage du visage et plus spécialement des joues et des paupières ainsi que des lèvres. Elle se présente notamment sous forme de fard à joues, à paupières, de blush, ou encore sous forme de poudre visage, corps ou déodorante. Elle peut se présenter compactée sous forme de stick, de coupelle ou de toute autre forme. Les poudres pour visage sont souvent destinées à matifier la peau et/ou à colorer la peau.

Les poudres de maquillage de la peau ou des lèvres des êtres humains contiennent généralement une grande quantité de poudres formées de pigments et/ou charges, liées entre elles à l'aide d'un liant généralement, liquide à température ambiante, du type huile, et éventuellement des additifs comme des actifs cosmétiques ou dermatologiques. Elles peuvent aussi contenir des tensioactifs.

Les poudres, lorsqu'elles sont appliquées sur la peau ou les lèvres, présentent l'inconvénient de transférer, c'est-à-dire de se déposer au moins en partie, en laissant des traces, sur certains supports avec lesquels elles peuvent être mises en contact, et notamment un vêtement ou la peau ou encore un verre, une tasse, une cigarette. Ce transfert intervient notamment, lors d'un frottement. Il s'ensuit une persistance médiocre du produit appliqué, nécessitant de renouveler régulièrement l'application de la poudre ou du fard. Par ailleurs, l'apparition de ces traces inacceptables notamment sur les cols de chemisier peut écarter certaines femmes de l'utilisation de ce type de maquillage.

De plus, ces poudres ont tendance à disparaître du visage ou du corps humain, du fait de la production de sueur et de sébum par la peau. Ainsi, on constate souvent une migration de la poudre dans les plis, rides et ridules de la peau faisant apparaître des stries ; ceci est notamment le cas dans les rides autour de l'oeil et les plis des paupières. Le maquillage devient alors disgracieux et hétérogène.

On obtient le même genre de phénomène avec les poudres pour visages ou blush ; dans ce cas la migration de la poudre se traduit par un marquage de certaines rides profondes, accusant l'âge de l'utilisatrice ainsi qu'une disparition de la couleur. Dans certains cas, on observe également une montée en brillance du maquillage au cours de la journée qui nécessite une autre application ou retouches ponctuelles de maquillage.

Pour absorber la sueur et le sébum, on utilise généralement des charges poreuses et éventuellement des liants fluorés "repoussant" le sébum et sueur et par exemple des dérivés de silicone fluorée (voir le document J09/020637) ou encore des charges et pigments enrobés par des fluorés (voir le document J08/295612). Ce type de poudre présente notamment les inconvénients d'être cher, ne permettant pas la commercialisation grand public. En outre, ces composés fluorés présentent souvent des problèmes d'innocuité et de toxicité.

Il subsiste donc le besoin d'une composition pulvérulente à application topique ne présentant pas les inconvénients ci-dessus, et ayant notamment des propriétés de "sans transfert" et de longue tenue, même lors d'une pression ou d'un frottement prononcé ou intensif. Cette composition permet, en outre, d'emprisonner les sécrétions produites par la peau, tout en limitant le dessèchement de la peau.

La demanderesse a constaté, de façon tout à fait surprenante, que l'utilisation d'un polymère dispersible dans une phase grasse, dans une composition cosmétique, dermatologique, pharmaceutique ou hygiénique permettait d'obtenir un maquillage de très bonne tenue, ne transférant pas du tout, résistant à l'eau, tout en étant agréable à l'application et à porter tout au long de la journée. En outre, la peau maquillée a un reflet soyeux, légèrement satiné tout à fait satisfaisant. Elle est non grasse et ne brille pas au cours du temps.

La présente invention a donc pour objet une composition pulvérulente à application topique, comprenant au moins un composé pulvérulent dont les particules sont liées entre elles par un liant, caractérisée par le fait que le liant comprend une phase grasse liquide et au moins 2 % en poids, par rapport au poids total de la composition, de polymère dispersible dans ladite phase grasse liquide.

Cette composition est en particulier une composition cosmétique, dermatologique, hygiénique ou pharmaceutique. Elle contient donc des ingrédients compatibles avec la peau, les muqueuses et les fibres kératiniques ou phanères.

En plus des avantages cités précédemment, la composition peut s'appliquer en une seule fois et conduit à un maquillage uniforme et homogène, ne migrant pas, résistant au frottement. En application sur les paupières, on n'observe pas ou pratiquement pas de stries. De plus la couvrance, le rendu des couleurs sur la peau, la douceur, le confort, et l'étalement de la composition sont supérieurs à ceux de l'art antérieur.

Sans pour autant être lié par ce qui suit, la demanderesse pense qu'une fois le solvant évaporé, les particules de polymère créent un dépôt de charges et/ou de pigments suffisamment cohésif pour maintenir le maquillage en place.

Le polymère utilisé dans la présente demande peut être de toute nature. On peut ainsi employer un polymère radicalaire, un polycondensat, voire un polymère d'origine naturelle et leurs mélanges. Le polymère peut être choisi par l'homme du métier en fonction de ses propriétés et selon l'application ultérieure souhaitée pour la composition. Ainsi, le polymère peut être filmifiable ou non. Cependant, pour une optimisation des propriétés "sans transfert", on préfère utiliser un polymère filmifiable.

L'invention a également pour objet une composition pulvérulente comprenant au moins un composé pulvérulent dont les particules sont liées entre elles par un liant, caractérisée par le fait que le liant comprend une phase grasse liquide volatile, cosmétique, dermatologique, hygiénique'ou pharmaceutique et au moins 2 % en poids, par rapport au poids total de la composition, de polymère filmifiable dispersible dans ladite phase grasse liquide.

Avantageusement, la composition de l'invention contient au moins un actif choisi parmi les actifs cosmétiques, dermatologiques, hygiéniques ou pharmaceutiques.

Un autre objet de l'invention est l'utilisation dans une composition pulvérulente cosmétique, dermatologique, pharmaceutique ou hygiénique, d'une phase grasse liquide contenant au moins 2 % en poids, par rapport au poids total de la composition, de polymère, notamment filmifiable, dispersible dans ladite phase grasse liquide comme liant et agent de diminution, voire suppression, du transfert de la composition déposée sur les lèvres et/ou la peau d'être humain vers un support mis en contact avec le dépôt, et/ou agent de diminution, voire suppression de la migration de ladite composition déposée et/ou agent d'augmentation de la tenue de ladite composition déposée.

Un autre objet de l'invention est l'utilisation dans d'une composition pulvérulente contenant au moins un composé pulvérulent dont les particules sont liées entre elles par un liant d'une phase grasse liquide volatile d'au moins 2 % en poids, par rapport au poids total de la composition, de polymère, notamment filmifiable, dispersible dans ladite phase grasse liquide, comme agent de diminution, voire suppression, du transfert et/ou de la migration et/ou comme agent d'augmentation de la tenue de la composition, déposée sur la peau et/ou les lèvres.

L'invention a encore pour objet un procédé de soin cosmétique ou de maquillage des lèvres ou de la peau d'humain, consistant à appliquer sur respectivement les lèvres ou la peau une composition cosmétique telle définie précédemment.

L'invention a encore pour objet un procédé cosmétique pour limiter, voire supprimer, le transfert d'une composition de maquillage ou de soin de la peau ou des lèvres sur un support différent de la dite peau et desdites lèvres, et/ou limiter, voire supprimer la migration et/ou augmenter la tenue de ladite composition contenant au moins un composé pulvérulent dont les particules sont liées entre elles par un liant, consistant à introduire dans la composition une phase grasse liquide contenant au moins 2 % en poids, par rapport au poids total de la composition d'un polymère dispersible dans ladite phase grasse liquide.

De façon avantageuse, le polymère se présente sous forme de particules dispersées et stabilisées en surface par au moins un stabilisant.

Un avantage de l'utilisation d'une dispersion de particules dans une composition de l'invention est que les particules restent à l'état de particules élémentaires, sans former d'agglomérats, dans la phase grasse, ce qui ne serait pas le cas avec des particules minérales de taille nanométrique. On obtient ainsi, un maquillage couvrant et uniforme.

Encore un autre avantage d'une telle dispersion est qu'il est possible de calibrer à volonté la taille des particules de polymère, et de moduler leur "polydispersité" en taille lors de la synthèse. Il est ainsi possible d'obtenir des particules de très petite taille, qui sont invisibles à l'oeil nu lorsqu'elles sont dans la composition et lorsqu'elles sont appliquées sur la peau ou les lèvres. Ceci ne serait pas possible avec des pigments sous forme particulaire, leur constitution ne permettant pas de moduler la taille moyenne des particules.

On a de plus constaté que les compositions selon l'invention, présentent des qualités d'étalement et d'adhésion sur la peau et les lèvres particulièrement intéressantes, ainsi qu'un toucher onctueux et agréable. Ces compositions ont, en outre, l'avantage de se démaquiller facilement notamment avec un lait démaquillant classique. Ceci est tout à fait remarquable puisque les compositions de l'art antérieur à propriétés "sans transfert" élevées sont très difficiles à démaquiller. En général, elles sont vendues avec un produit démaquillant spécifique, ce qui introduit une contrainte supplémentaire pour l'utilisatrice.

Les compositions selon l'invention comprennent donc avantageusement comme liant de composé pulvérulent une dispersion stable de particules généralement sphériques d'au moins un polymère, dans une phase grasse liquide physiologiquement acceptable. Ces dispersions peuvent notamment se présenter sous forme de nanoparticules de polymères en dispersion stable dans ladite phase grasse. Les nanoparticules sont de préférence d'une taille comprise entre 5 et 600 nm, étant donné qu'au-delà d'environ 600 nm, les dispersions de particules deviennent beaucoup moins stables.

Encore un avantage de la dispersion de polymère de la composition de l'invention est la possibilité de faire varier la température de transition vitreuse (Tg) du polymère ou du système polymérique (polymère plus additif du type plastifiant), et de passer ainsi d'un polymère mou à un polymère plus ou moins dur, permettant de régler les propriétés physiques de la compositions en fonction de l'application envisagée.

Il est possible d'utiliser des polymères filmifiables, de préférence ayant une (Tg) basse, inférieure ou égale à la température de la peau. On obtient ainsi une dispersion qui peut filmifier lorsqu'elle est appliquée sur un support, ce qui n'est pas le cas lorsque l'on utilise des dispersions de pigments minéraux selon l'art antérieur.

Les polymères utilisables dans la composition de l'invention ont de préférence un poids moléculaire de l'ordre de 2000 à 10 000 000 et une (Tg) de -100°C à 300°C.

Lorsque le polymère présente une température de transition vitreuse trop élevee pour l'application souhaitée, on peut lui associer un plastifiant de manière à abaisser cette température du mélange utilisé. Le plastifiant peut être choisi parmi les plastifiants usuellement utilisés dans le domaine d'application et notamment parmi les composés susceptibles d'être des solvants du polymère.

Parmi les polymères filmifiables, on peut citer des homopolymères ou des copolymères radicalaires, acryliques ou vinyliques, de préférence ayant une Tg inférieure ou égale à 40°C et notamment les acrylates de méthyle éventuellement copolymérisés avec l'acide acrylique.

Parmi les polymères non filmifiables, on peut citer des homopolymères ou copolymères radicalaires, vinyliques ou acryliques, éventuellement réticulés, ayant de préférence une Tg supérieure ou égale à 40°C, tels que le polyméthacrylate de méthyle, le polystyrène ou le polyacrylate de tertiobutyle.

De façon non limitative, les polymères de l'invention peuvent être choisis parmi, les polymères ou copolymères suivants : polyuréthannes, polyuréthannes-acryliques, polyurées, polyurée-polyuréthannes, polyester-polyuréthannes, polyéther-polyuréthannes, polyesters, polyesters amides, polyesters à chaîne grasse, alkydes ; polymères ou copolymères acryliques et/ou vinyliques ; copolymères acryliques-silicone ; polyacrylamides ; polymères siliconés et leurs mélanges.

Le phase grasse liquide dans laquelle est dispersé le polymère, peut être constituée de toute huile cosmétiquement ou dermatologiquement acceptable, notamment choisie parmi les huiles d'origine minérale, animale, végétale ou synthétique, carbonées, hydrocarbonées et/ou siliconées, seules ou en mélange dans la mesure où elles forment un mélange homogène et stable et où elles sont compatibles avec l'utilisation envisagée.

Par "phase grasse liquide", on entend tout milieu non aqueux liquide à température ambiante. Par "phase grasse liquide volatile", on entend tout milieu non aqueux susceptible de s'évaporer de la peau et/ou des lèvres, à température ambiante en moins d'une heure.

On peut ainsi citer les huiles hydrocarbonées telles que l'huile de paraffine ou de vaseline, l'huile de vison, de tortue, de soja, le perhydrosqualène, l'huile d'amande douce, de calophyllum, de palme, de pépins de raisin, de sésame, de maïs, de parléam, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les polydiméthylsiloxane (PDMS), éventuellement phénylées telles que les phényltriméthicones, ou éventuellement substitués par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes.

Avantageusement, on peut utiliser une ou plusieurs huiles volatiles à température ambiante. Ces huiles volatiles sont favorables à l'obtention d'un dépôt cohésif à propriétés "sans transfert" total. Après évaporation de ces huiles, on obtient un dépôt filmogène non cohésif de particules, non collant sur la peau ou les muqueuses, suivant respectivement le mouvements de la peau ou des lèvres, sur lesquelles la composition est appliquée. Ces huiles volatiles facilitent, en outre, l'application de la composition sur la peau et les lèvres.

Ces huiles peuvent être des huiles hydrocarbonées, des huiles silicones comportant éventuellement des groupements alkyle ou alkoxy en bout de chaîne siliconée ou pendante.

Comme huile de silicone volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone ainsi que les isoparaffines en $C_8$-$C_{16}$. Ces huiles volatiles représentent notamment une quantité comprise entre 0,5 et 30 % du poids total de la composition, et mieux de 1 à 20 %.

Comme huile volatile utilisable dans l'invention, on peut citer notamment l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane ou les isoparaffines en $C_8$-$C_{16}$ telles que les 'ISOPARs', les PERMETYLs et notamment l'isododécane.

Dans un mode particulier de réalisation de l'invention, on choisit le phase grasse liquide dans le groupe comprenant :

- les composés liquides non aqueux ayant un paramètre de solubilité global selon l'espace de solubilité de HANSEN inférieur à 17 (MPa)$^{1/2}$,
- ou les monoalcools ayant un paramètre de solubilité global selon l'espace de solubilité de HANSEN inférieur ou égal à 20 (MPa)$^{1/2}$,
- ou leurs mélanges.

Le paramètre de solubilité global $\delta$ global selon l'espace de solubilité de HANSEN est défini dans l'article "Solubility parameter values" de Eric A. Grulke de l'ouvrage "Polymer Handbook" 3$^{ème}$ édition, Chapitre VII, pages 519-559 par la relation :

$$\delta = (d_D{}^2 + d_P{}^2 + d_H{}^2)^{1/2}$$

dans laquelle

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc.). La définition des solvants dans l'espace de solubilité tridimensionnel selon HANSEN est décrite dans l'article de C. M. HANSEN. "The three dimensional solubility parameters" J. Paint Technol. 39, 105 (1967).

Parmi les phases grasses liquides ayant un paramètre de solubilité global selon l'espace de solubilité de HANSEN inférieur ou égal à 17 (MPa)$^{1/2}$, on peut citer des huiles végétales formées par des esters d'acides gras et de polyols, en particulier les triglycérides, telles que l'huile de tournesol, de sésame ou de colza, ou les esters dérivés d'acides ou d'alcools à longue chaîne (c'est à dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle. On peut également citer les hydrocarbures et notamment des huiles de paraffine, de vaseline, ou le polyisobutylène hydrogéné, l'isododécane, ou encore les 'ISOPARs', isoparaffines volatiles. On peut encore citer les huiles de silicone telles que les polydiméthylsiloxanes et les polyméthylphénylsiloxanes, éventuellement substitués par des groupements aliphatiques et/ou aromatiques, ou par des groupements fonctionnels tels que des groupements hydroxyle, thiol et/ou amine, et les huiles siliconées volatiles, notamment cycliques. On peut également citer les solvants, seuls ou en mélange, choisis parmi (i) les esters linéaires, ramifiés ou cycliques, ayant plus de 6 atomes de carbone, (ii) les éthers ayant plus de 6 atomes de carbone, (iii) les cétones ayant plus de 6 atomes de carbone. Par monoalcools ayant un paramètre de solubilité global selon l'espace de solubilité de HANSEN inférieur ou égal à 20 (MPa)$^{1/2}$, on entend les alcools gras aliphatiques ayant au moins 6 atomes de carbone, la chaîne hydrocarbonée ne comportant pas de groupement de substitution. Comme monoalcools selon l'invention, on peut citer l'alcool oléique, le décanol, le dodécanol, l'octadécanol et l'alcool linoléique.

Comme milieu non aqueux, on peut aussi utiliser ceux décrits dans le document FR-A-2 710 646 de L.V.M.H.

Le choix du milieu non aqueux est effectué par l'homme du métier en fonction de la nature des monomères constituant le polymère et/ou de la nature du stabilisant, comme indiqué ci-après.

De plus, la phase grasse liquide dans laquelle est dispersé le polymère peut représenter de 0,5 à 30 % du poids total de la composition et de préférence de 1 à 25 %.

La dispersion de polymère peut être fabriquée comme décrit dans le document EP-A-749747. La polymérisation peut être effectuée en dispersion, c'est-à-dire par précipitation du polymère en cours de formation, avec protection des particules formées avec un stabilisant.

On prépare donc un mélange comprenant les monomères initiaux ainsi qu'un amorceur radicalaire. Ce mélange est dissous dans un solvant appelé, dans la suite de la présente description, "solvant de synthèse". Lorsque la phase grasse est une huile non volatile, on peut effectuer la polymérisation dans un solvant organique apolaire (solvant de synthèse) puis ajouter l'huile non volatile (qui doit être miscible avec ledit solvant de synthèse) et distiller sélectivement le solvant de synthèse.

On choisit donc un solvant de synthèse tel que les monomères initiaux, et l'amorceur radicalaire, y sont solubles, et les particules de polymère obtenu y sont insolubles afin qu'elles y précipitent lors de leur formation. En particulier, on peut choisir le solvant de synthèse parmi les alcanes tels que l'heptane, l'isododécane ou le cyclohexane.

Lorsque la phase grasse choisie est une huile volatile, on peut directement effectuer la polymérisation dans ladite huile qui joue donc également le rôle de solvant de synthèse. Les monomères doivent également y être solubles, ainsi que l'amorceur radicalaire, et le polymère obtenu doit y être insoluble.

Les monomères sont de préférence présents dans le solvant de synthèse, avant polymérisation, à raison de 5-20% en poids du mélange réactionnel. La totalité des monomères peut être présente dans le solvant avant le début de la réaction, ou une partie des monomères peut être ajoutée au fur et à mesure de l'évolution de la réaction de polymérisation.

L'amorceur radicalaire peut être notamment l'azo-bis-isobutyronitrile ou le tertiobutylperoxy-2-éthyl hexanoate.

Les particules de polymère sont stabilisées en surface, au fur et à mesure de la polymérisation, grâce à un stabilisant qui peut être un polymère séquencé, un polymère greffé, et/ou un polymère statistique, seul ou en mélange. La stabilisation peut être effectuée par tout moyen connu, et en particulier par ajout direct du polymère séquencé, polymère greffé et/ou polymère statistique, lors de la polymérisation.

Le stabilisant est de préférence également présent dans le mélange avant polymérisation. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée ; les polymères hydrocarbonés greffés avec une chaîne siliconée.

Conviennent également les copolymères greffés ayant par exemple un squelette insoluble de type polyacrylique avec des greffons solubles de type acide polyhydroxystéarique : copolymères à base d'acrylates ou de méthacrylates d'alcools en $C_1$-$C_4$, et d'acrylates ou de méthacrylates d'alcools en $C_8$-$C_{30}$.

Comme copolymères blocs greffés ou séquencés comprenant au moins un bloc de type polyorganosiloxane et au moins un bloc d'un polymère radicalaire, on peut citer les copolymères greffés de type acrylique/silicone qui peuvent être employés notamment lorsque le milieu non aqueux est siliconé.

Comme copolymères blocs greffés ou séquencés comprenant au moins un bloc de type polyorganosiloxane et au moins d'un polyéther, on peut utiliser les copolyol diméthicones tels que ceux vendu sous la dénomination "DOW CORNING 3225C" par la société DOW CORNING, les lauryl méthicones tels que ceux vendu sous la dénomination "DOW CORNING Q2-5200 par la société "DOW CORNING".

Comme copolymères blocs greffés ou séquencés comprenant au moins un bloc résultant de la polymérisation de diène, hydrogéné ou non hydrogéné, et au moins un bloc d'un polymère vinylique, on peut citer les copolymères séquencés, notamment de type "dibloc" ou "tribloc" du type polystyrène/polyisoprène, polystyrène/polybutadiène tels que ceux vendus sous le nom de 'LUVITOL HSB' par BASF, du type polystyrène/copoly(éthylènepropylène) tels que ceux vendus sous le nom de 'KRATON' par Shell Chemical Co ou encore du type polystyrène/copoly(éthylène-butylène).

Comme copolymères blocs greffés ou séquencés comprenant au moins un bloc résultant de la polymérisation de diène, hydrogéné ou non hydrogéné, et au moins un bloc d'un polymère acrylique, on peut citer les copolymères bi- ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation de diènes, hydrogéné ou non hydrogéné, et au moins un bloc d'un polyéther, on peut citer les copolymères bi- ou triséquencés polyoxyéthylène/polybutadiène ou polyoxyéthylène/polyisobutylène.

Lorsqu'on utilise un polymère statistique en tant que stabilisant, on le choisit de manière à ce qu'il possède une quantité suffisante de groupements le rendant soluble dans le solvant de synthèse envisagé.

On peut ainsi employer des copolymères d'acrylates ou de méthacrylates d'alcools en $C_1$-$C_4$, et d'acrylates ou de méthacrylates d'alcools en $C_8$-$C_{30}$. On peut en particulier citer le copolymère méthacrylate de stéaryle/méthacrylate de méthyle.

De préférence, on choisit en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

D'autre part, lorsque le phase grasse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester.

Lorsque le phase grasse liquide ne comprend pas d'huile de silicone, l'agent stabilisant est de préférence choisi dans le groupe constitué par :

- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alcools en $C_1$-$C_4$, et d'acrylates ou de méthacrylates d'alcools en $C_8$-$C_{30}$,

- (c) les copolymères blocs greffés ou séquencés comprenant au moins un bloc résultant de la polymérisation de diène, hydrogéné ou non hydrogéné,

et au moins un bloc d'un polymère vinylique ou acrylique ou d'un polyéther ou d'un polyester, ou leurs mélanges.

Les dispersions obtenues selon l'invention peuvent alors être utilisées dans une composition notamment cosmétique, dermatologique, pharmaceutique et/ou hygiénique, ou de façon générale physiologique, telle qu'une composition de soin ou de maquillage de la peau ou des lèvres, ou encore une composition capillaire ou une composition solaire ou de coloration de la peau.

Suivant l'application, on pourra choisir d'utiliser des dispersions de polymères filmifiables ou non filmifiables, dans des huiles volatiles ou non volatiles. La présence d'une faible quantité, par exemple de l'ordre de 0,1 à 10 % d'huile non volatile à température ambiante permet d'augmenter la cohésion des différents composés pulvérulents.

La composition peut comprendre un ou plusieurs composés pulvérulents, par exemple à raison de 68 à 96,8 % du poids total de la composition. Les composés pulvérulents peuvent être choisis parmi les pigments et/ou les nacres et/ou les charges habituellement utilisés dans les compositions cosmétiques ou dermatologiques. Avantageusement, les composés pulvérulents représentent de 75 à 95 % du poids total de la composition. Plus la quantité de composés pulvérulents diminue, plus les qualités de sans transfert et de tenue augmentent.

Par ailleurs, la propriété de sans transfert augmente avec la quantité de polymère dispersible dans la phase grasse liquide. En pratique, le polymère peut représenter en matière active jusqu'à 20 % (en matière active ou sèche) du poids total de la composition. En utilisant au-dessus de 10 % en poids de matière active de polymère dans la composition et jusqu'à 20 %, on obtient un dépôt sans transfert et d'excellente tenue. Entre 2 % et 10 % l'effet sans transfert et de tenue sont notables. On peut donc adapter les propriétés sans transfert à volonté, ce qui n'était pas possible avec les compositions sans transfert de l'art antérieur, et ce, sans nuire au confort du maquillage déposé.

La composition de l'invention peut comprendre avantageusement moins de 30 % en poids de phase grasse liquide. Au dessus de 30 %, on obtient une texture crème, non pulvérulente.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse; le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon (Orgasol de chez Atochem ou Nylon 12), de poly-β-alanine et de polyéthylène, le Téflon, l'oxychlorure de bismuth, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut, en outre contenir des colorants solubles dans la phase grasse de la composition. Ces colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter de 0,01 à 20 % du poids total de la compositions et mieux de 0,1 à 6 %.

Le polymère de la composition de l'invention permet la formation d'un dépôt cohésif sur les lèvres et/ou la peau, formant un réseau piégeant les pigments et/ou les actifs ainsi que les colorants liposolubles (si présents). Selon la quantité relative de polymère et de pigments, utilisée, il est possible d'obtenir un dépôt plus ou moins mat ou satiné et plus ou moins sans transfert et/ou de longue tenue.

Comme actifs cosmétiques, dermatologiques, hygiéniques ou pharmaceutiques, utilisables dans la composition de l'invention, on peut citer les hydratants, vitamines, acides gras essentiels, sphingolipides, filtres solaires. Ces actifs sont présents en quantité généralement utilisée dans les poudres. En particulier, ils sont présents à raison de 0,001 à 20 % du poids total de la composition.

La composition selon l'invention peut, de plus, comprendre, selon le type d'application envisagée, les constituants classiquement utilisées dans les domaines considérés, qui sont présents en une quantité appropriée à la forme galénique souhaitée.

En particulier, elle peut comprendre, outre, la phase grasse liquide dans laquelle le polymère est stabilisé des phases grasses additionnelles qui peuvent être choisies parmi les cires, les gommes et/ou les corps gras pâteux, d'origine végétale, animale, minérale ou de synthèse, voire siliconé, et leurs mélanges.

Parmi les cires solides à température ambiante, susceptibles d'être présentes dans la composition selon l'invention, on peut citer les cires hydrocarbonées telles que la cire d'abeilles, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène, les cires obtenues par synthèse de Fischer-Tropsch, les huiles hydrogénées, les esters gras et les glycérides concrets à 25°C. On peut également utiliser des cires de silicone, parmi lesquelles on peut citer les alkyl, alcoxy et/ou esters de polyméthylsiloxane. Les cires peuvent se présenter sous forme de dispersions stables de particules colloïdales de cire telles qu'elles peuvent être préparées selon des méthodes connues, telles que celles de "Microemulsions Theory and Practice", L. M. Prince Ed., Academic Press (1977), pages 21-32. Comme cire liquide à température ambiante, on peut citer l'huile de Jojoba.

Les cires peuvent être présentes à raison de 0-28 % du poids total de la composition et mieux de 1 à 10 %.

La composition peut comprendre, en outre, tout additif usuellement utilisé dans de telles compositions, tel que des épaississants, des antioxydants, des parfums, des conservateurs, des tensioactifs, des polymères liposolubles comme les polyalkylènes, notamment le polybutène, les polyacrylates et les polymères siliconés compatibles avec la phase grasse ainsi que les copolymères de polyvinylpyrolidone. Bien entendu l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

Dans un mode de réalisation particulier de l'invention, les compositions selon l'invention peuvent être préparées de manière usuelle par l'homme du métier. Elles peuvent se présenter sous forme d'une poudre pressée ou compactée, par exemple sous forme d'un stick ou de coupelle utilisable par prise au doigt ou à l'éponge : en particulier, elles trouvent une application en tant que blush, fard à joues ou à paupières, poudre à lèvres, poudre de soin ou de maquillage du visage ou du corps ou encore de poudre déodorante. Elles peuvent aussi se présenter sous forme d'une poudre libre, notamment comme poudre à joues, de soin ou de maquillage du visage ou du corps ou de poudre déodorante.

Les compositions de l'invention sont avantageusement anhydres, et peuvent contenir moins de 5 % d'eau par rapport au poids total de la composition. Elles peuvent alors se présenter notamment sous forme d'émulsion solide'à toucher poudreux. Elles peuvent aussi contenir des vésicules contenant des lipides ioniques et/ou non ioniques. Ces formes galéniques sont préparées selon les méthodes usuelles des domaines considérés.

Ces compositions à application topique peuvent constituer notamment une composition cosmétique, dermatologique, hygiénique ou pharmaceutique de protection, de traitement ou de soin pour le visage, pour le cou, pour les mains ou pour le corps (par exemple poudre de soin anhydre parfumée ou non, poudre solaire), une composition de maquillage (par exemple gel de maquillage) ou une composition de bronzage artificiel.

L'invention est illustrée plus en détail dans les exemples suivants.

## Exemple 1 de dispersion de polymère

On prépare une dispersion de copolymère non réticulé d'acrylate de méthyle et d'acide acrylique dans un rapport 85/15, dans de l'isododécane, selon la méthode de l'exemple 1 du document EP-A-749 746, en remplaçant l'heptane par de l'isododécane. On obtient ainsi une dispersion de particules de poly(acrylate de méthyle/acide acrylique) stabilisées en surface dans de l'isododécane par un copolymère dibloc séquencé polystyrène/copoly(éhylène-propylène) vendu sous le nom de KRATON G1701 (Shell), ayant un taux de matière sèche de 22,6% en poids et une taille moyenne des particules de 175 nm (polydispersité : 0,05) et une Tg de 20°C. Ce copolymère est filmifiable.

## Exemple 2 de dispersion de polymère

On prépare une dispersion de polyméthacrylate de méthyle réticulé par du diméthacrylate d'éthylène glycol, dans de l'isododécane, selon la méthode de l'exemple 2 du document EP-A-749 746, en remplaçant par L'ISOPAR L de l'isododécane. On obtient ainsi une dispersion de particules de polyméthacrylate de méthyle stabilisées en surface dans de l'isododécane par un copolymère dibloc séquencé polystyrène/copoly(éhylène-propylène) vendu sous le nom de KRATON G1701 (Shell), ayant un taux de matière sèche de 19,7% en poids et une taille moyenne des particules de 135 nm (polydispersité : 0,05) et une Tg de 100°C. Ce copolymère est non filmifiable à température ambiante.

## Exemple 3 de fard à paupières

On prépare un fard à paupières ayant la composition suivante :

.    dispersion selon l'exemple 1 (3,4 % de polymère) 15,0 g
.    talc        29,0 g

- mica     20,0 g
- oxychlorure de bismuth     8,0 g
- stéarate de zinc     3,0 g
- Nylon 12     20,0 g
- pigments     5 g

Les pigments renferme un mélange de DC Red 27, DC Red 7, DC Red 36, d'oxyde de fer noir et d'oxyde de fer brun. La composition est préparée par prémélange des charges et des pigments dans un malaxeur du type Lôdige, puis incorporation du liant dans ce prémélange et homogénéisation delà même façon. Le tout est ensuite broyé dans un broyeur Alpine de la société Osokawa. La poudre obtenue est tamisée et compactée dans des coupelles.

**Exemple 4 de fard à paupières**

On prépare un fard à paupières comme dans l'exemple 3 ayant la composition suivante :

- dispersion selon l'exemple 1     6,6 g
- talc     22,9 g
- mica     22,0 g
- oxychlorure de bismuth     8,0 g
- stéarate de zinc     3,0 g
- Nylon 12     20,0 g
- oxyde de fer jaune     3,0 g
- oxyde de fer rouge     6,5 g
- oxyde de fer noir     6,0 g
- dioxyde de titane     2,0 g

Le broyeur est ici un broyeur à jet d'air.

**Exemple 5 de fard à paupières**

On prépare un fard à paupières comme dans l'exemple 4, en remplaçant la dispersion de polymère par celle obtenue selon l'exemple 2.

**Contre exemple de fard à paupières**

On prépare un fard à paupières selon l'art antérieur contenant la même composition que celle des exemples 4 et 5, à l'exception de la dispersion de polymère qui est remplacée par un mélange d'huiles carbonées contenant :

- 3,6 g de triglycérides d'acide caprique/caprylique,
- 2,0 g d'isoparaffine hydrogénée (non volatile),
- 1,0 g d'huile de jojoba

**Tests comparatifs**

Un premier panel de 10 personnes spécialisées a appliqué par demi-visage sur les paupières, et en aveugle, le fard de l'exemple 4 et celui du contre exemple. Des notes de 1 à 10 ont été données pour chacun des produits. Le résultat de ce test est donné ci-après :

- Tenue après 4 h. :     6,6 pour l'invention contre 5,4 pour le contre exemple,
- Stabilité couleur après 4 h. :     6,5 pour l'invention contre 5,9 pour le contre exemple,
- Prise du produit :     7 pour l'invention contre 6,3 pour le contre exemple,
- Adhérence sur la paupière :     6,9 pour l'invention contre 5,9 pour le contre exemple,
- Facilité d'étalement :     7,2 pour l'invention contre 5,6 pour le contre exemple,
- Douceur :     6,7 pour l'invention contre 5,8 pour le contre exemple,
- Uniformité :     6,3 pour l'invention contre 5,1 pour le contre exemple,
- Couleur fidèle au compact :     7,3 pour l'invention contre 6,2 pour le contre exemple,
- Couvrance :     6,7 pour l'invention contre 5,1 pour le contre exemple,
- Confort :     7,7 pour l'invention contre 7,3 pour le contre exemple.

Un second panel de spécialistes a testé, par demi-visage sur les paupières et en aveugle le fard de l'exemple 5 en comparaison de celui du contre-exemple. L'adhérence sur la paupière, la douceur à l'application, l'uniformité du maquillage et la couvrance du fard selon l'invention sont supérieurs à celles du fard classique.

**Exemple 6 de fard à paupières**

On prépare un fard à paupières comme dans l'exemple 3 ayant la composition suivante :

- dispersion selon l'exemple 1     10,0 g
- talc     19,5 g
- mica     22,0 g
- oxychlorure de bismuth     8,0 g
- stéarate de zinc     3,0 g
- Nylon 12     20,0 g
- oxyde de fer jaune     3,0 g
- oxyde de fer rouge     6,5 g
- oxyde de fer noir     6,0 g
- dioxyde de titane     2,0 g

Le broyeur est ici un broyeur à jet d'air.

**Revendications**

1. Composition pulvérulente à application topique, comprenant au moins un composé pulvérulent dont les particules sont liées entre elles par un liant, caractérisée par le fait que le liant comprend une phase grasse liquide et au moins 2 % en poids, par rapport au poids total de la composition, de polymère dispersible dans ladite phase grasse liquide.

2. Composition selon la revendication 1, caractérisée en ce que le polymère est filmifiable.

3. Composition pulvérulente comprenant au moins un composé pulvérulent dont les particules sont liées entre elles par un liant, caractérisée par le fait que le liant comprend une phase grasse liquide volatile, cosmétique, dermatologique, hygiénique ou pharmaceutique et au moins 2 % en poids, par rapport au poids total de la composition, de polymère filmifiable dispersible dans ladite phase grasse liquide.

4. Composition selon l'une des revendications 1 à 3, comprenant, en outre, au moins un actif choisi parmi les actifs cosmétiques, dermatologiques, hygiéniques ou pharmaceutiques.

5. Composition selon l'une des revendications 2 à 4, comprenant, en outre, au moins une matière colorante.

6. Composition selon l'une des revendications précédentes, dans laquelle le polymère se présente sous forme de particules dispersées et stabilisées en surface par au moins un stabilisant.

7. Composition selon l'une des revendications précédentes, dans laquelle le polymère est choisi parmi les polymères radicalaires, les polycondensats, les polymères d'origine naturelle et leurs mélanges.

8. Composition selon l'une des revendications précédentes, dans laquelle le polymère est choisi parmi les polyuréthannes, polyuréthannes-acryliques, polyurées, polyurée/polyuréthannes, polyester-polyuréthannes, polyéther-polyuréthannes, polyesters, polyesters amides, polyesters à chaîne grasse, alkydes ; polymères ou copolymères acryliques et/ou vinyliques ; copolymères acryliques-silicone ; polyacrylamides ; polymères siliconés et leurs mélanges.

9. Composition selon l'une des revendications précédentes, dans laquelle la phase grasse liquide est constitué d'huiles d'origine minérale, animale, végétale ou synthétique, carbonées, hydrocarbonées et/ou siliconées, seules ou en mélange.

10. Composition selon l'une des revendications précédentes, dans laquelle la phase grasse liquide est choisie parmi l'huile de paraffine ou de vaseline, l'huile de vison, de tortue, de soja, le perhydrosqualène, l'huile d'amande douce, de calophyllum, de palme, de parléam, de pépins de raisin, de sésame, de maïs, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les PDMS éventuellement phénylés tels que les phényltriméthicones ou éventuellement substitués par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes ; les huiles volatiles telles que l'octaméthylcyclotétrasiloxane, le décaméthylcyclopentasiloxane, l'hexadéméthylcyclohexasiloxane, l'heptaméthylhexyltrisiloxane, l'heptaméthyloctyltrisiloxane ou les isoparaffines en $C_8$-$C_{16}$, et notamment l'isododécane.

11. Composition selon l'une des revendications précédentes, dans laquelle la phase grasse liquide est choisie dans le groupe comprenant:

- les composés liquides non aqueux ayant un paramètre de solubilité global selon l'espace de solubilité de HANSEN inférieur à 17 $(MPa)^{1/2}$,
- ou les monoalcools ayant un paramètre de solubilité global selon l'espace de solubilité de HANSEN inférieur ou égal à 20 $(MPa)^{1/2}$,
- ou leurs mélanges.

12. Composition selon l'une des revendications 1, 2 et 4 à 11, caractérisée en ce que la phase grasse contient au moins une huile volatile à température ambiante.

13. Composition selon l'une des revendications précédentes, dans laquelle la phase grasse liquide contient au moins une huile non volatile.

14. Composition selon l'une des revendications 6 à 13, dans laquelle le stabilisant est choisi parmi les polymères séquencés, les polymères greffés, les polymères statistiques et leurs mélanges.

**15.** Composition selon la revendication 14, dans laquelle le stabilisant est choisi parmi les polymères siliconés greffés avec une chaîne hydrocarbonée ; les polymères hydrocarbonés greffés avec une chaîne siliconée ; les copolymères greffés ayant un squelette insoluble de type polyacrylique avec des greffons solubles de type acide polyhydroxystéarique ; les copolymères blocs greffés ou séquencés comprenant au moins un bloc de type polyorganosiloxane et au moins un bloc d'un polymère radicalaire ; les copolymères blocs greffés ou séquencés comprenant au moins un bloc de type polyorganosiloxane et au moins d'un polyéther ; les copolymères d'acrylates ou de méthacrylates d'alcools en $C_1$-$C_4$, ou d'acrylates ou de méthacrylates d'alcools en $C_8$-$C_{30}$; les copolymères blocs greffés ou séquencés comprenant au moins un bloc résultant de la polymérisation de diène et au moins un bloc d'un polymère vinylique ; les copolymères blocs greffés ou séquencés comprenant au moins un bloc résultant de la polymérisation de diènes et au moins un bloc d'un polymère acrylique ; les copolymères blocs greffés ou séquencés comprenant au moins un bloc résultant de la polymérisation de diènes et au moins un bloc d'un polyéther.

**16.** Composition selon la revendication 14 ou 15, caractérisée en ce que le stabilisant est un polymère bloc greffé ou séquencé, comprenant au moins un bloc résultant de la polymérisation de diène et au moins un bloc d'un polymère vinylique.

**17.** Composition selon l'une des revendications précédentes, comprenant, en outre, au moins une phase grasse additionnelle choisie parmi les cires, les gommes et/ou les corps gras pâteux, d'origine végétale, animale, minérale, de synthèse, ou siliconés et leurs mélanges.

**18.** Composition selon l'une des revendications 1, 5 à 16 caractérisée en ce que le composé pulvérulent est choisi parmi les charges, les pigments, les nacres et leurs mélanges.

**19.** Composition selon l'une des revendications 1 à 18, caractérisée en ce que le composé pulvérulent représente jusqu'à 96,8 % du poids total de la composition.

**20.** Composition selon l'une des revendications 13 à 19, caractérisée en ce que l'huile non volatile représentent de 0,1 à 10 % du poids total de la composition.

**21.** Composition selon l'une des revendications précédentes, caractérisée en ce que le polymère représente en matière sèche jusqu'à 20 % du poids total de la composition.

**22.** Composition selon l'une des revendications précédentes, caractérisée en ce que le polymère représente en matière sèche de 2 à 10 % du poids total de la composition.

**23.** Composition selon l'une des revendications précédentes, caractérisée en ce que la phase grasse liquide contient au moins une huile choisie parmi les isoparaffines en $C_8$-$C_{16}$ et les silicones linéaires ou cycliques ayant de 2 à 7 atomes de silicium, ces silicones comportant éventuellement des groupes alkyle ayant de 1 à 10 atomes de carbone, leurs mélanges.

**24.** Composition selon l'une des revendications précédentes, se présentant sous forme d'une poudre compactée, pressée ou libre.

**25.** Composition selon l'une des revendications précédentes, se présentant sous forme anhydre.

**26.** Composition selon l'une des revendications précédentes, se présentant sous la forme d'un produit de soin et/ou de maquillage de la peau et/ou des lèvres.

**27.** Composition selon l'une des revendications précédentes, se présentant sous forme d'un fard à joues ou à paupières, d'un produit à lèvres, d'une poudre de soin ou de maquillage du visage ou du corps, d'une.poudre déodorante ou de blush.

**28.** Utilisation dans une composition pulvérulente cosmétique, dermatologique, pharmaceutique ou hygiénique, d'une phase grasse liquide contenant au moins 2 % en poids, par rapport au poids total de la composition, de polymère dispersible dans ladite phase grasse liquide, comme liant et agent de diminution, voire suppression, du transfert de la composition déposée sur les lèvres et/ou la peau d'être humain vers un support mis en contact avec le dépôt.

**29.** Utilisation dans une composition pulvérulente cosmétique, dermatologique, pharmaceutique ou hygiénique, d'une phase grasse liquide contenant au moins 2 % en poids, par rapport au poids total de la composition, de polymère dispersible dans ladite phase grasse liquide, comme liant et agent de diminution, voire suppression, de la migration de la composition déposée sur les lèvres et/ou la peau d'être humain, dans les plis, rides et ridules de la peau.

**30.** Utilisation dans une composition pulvérulente cosmétique, dermatologique, pharmaceutique ou hygiénique, d'une phase grasse liquide contenant au moins 2 % en poids, par rapport au poids total de la composition, de polymère dispersible dans ladite phase grasse liquide, comme liant et agent d'augmentation de la tenue.

31. Utilisation dans une composition pulvérulente contenant au moins un composé pulvérulent dont les particules sont liées entre elles par un liant, d'une phase grasse liquide volatile et d'au moins 2 % en poids, par rapport au poids total de la composition, de polymère dispersible dans ladite phase grasse liquide, comme agent de diminution, voire suppression, du transfert de la composition déposée sur la peau et/ou les lèvres.

32. Utilisation dans une composition pulvérulente contenant au moins un composé pulvérulent dont les particules sont liées entre elles par un liant, d'une phase grasse liquide volatile et d'au moins 2 % en poids, par rapport au poids total de la composition, de polymère dispersible dans ladite phase grasse liquide, comme agent de diminution, voire suppression de la migration de la composition déposée sur la peau et/ou les lèvres.

33. Utilisation dans une composition pulvérulente contenant au moins un composé pulvérulent dont les particules sont liées entre elles par un liant, d'une phase grasse liquide volatile et d'au moins 2 % en poids, par rapport au poids total de la composition, de polymère dispersible dans ladite phase grasse liquide, comme agent d'augmentation de la tenue de la composition déposée sur la peau et/ou les lèvres.

34. Utilisation selon l'une quelconque des revendications 28 à 33, caractérisée en ce que le polymère est filmifiable.

35. Procédé de soin cosmétique ou de maquillage des lèvres ou de la peau, consistant à appliquer sur respectivement les lèvres ou la peau une composition cosmétique telle définie aux revendications 1 à 27.

36. Procédé cosmétique pour limiter, voire supprimer, le transfert d'une composition de maquillage ou de soin de la peau ou des lèvres sur un support différent de la peau ou des lèvres et/ou limiter, voire supprimer la migration et/ou augmenter la tenue de ladite composition, la composition contenant au moins un composé pulvérulent dont les particules sont liées entre elles par un liant, consistant à introduire dans la composition une phase grasse liquide contenant au moins 2 % en poids, par rapport au poids total de la composition d'un polymère dispersible dans ladite phase grasse liquide.

**Claims**

1. Pulverulent composition for topical application comprising at least one pulverulent compound, the particles of which are bonded to one another by a binder, characterized in that the binder comprises a liquid fatty phase and at least 2% by weight, with respect to the total weight of the composition, of polymer which is dispersible in the said liquid fatty phase.

2. Composition according to Claim 1, characterized in that the polymer can form a film.

3. Pulverulent composition comprising at least one pulverulent compound, the particles of which are bonded to one another by a binder, characterized in that the binder comprises a cosmetic, dermatological, hygiene or pharmaceutical volatile liquid fatty phase and at least 2% by weight, with respect to the total weight of the composition, of polymer, which can form a film, which is dispersible in the said liquid fatty phase.

4. Composition according to one of Claims 1 to 3, additionally comprising at least one active principle chosen from cosmetic, dermatological, hygiene or pharmaceutical active principles.

5. Composition according to one of Claims 2 to 4, additionally comprising at least one colouring material.

6. Composition according to one of the preceding claims, in which the polymer is provided in the form of dispersed particles which are stabilized at the surface by at least one stabilizing agent.

7. Composition according to one of the preceding claims, in which the polymer is chosen from radical polymers, polycondensates, polymers of natural origin and their mixtures.

8. Composition according to one of the preceding claims, in which the polymer is chosen from polyurethanes, polyurethane-acrylics, polyureas, polyureapolyurethanes, polyester-polyurethanes, polyether-polyurethanes, polyesters, polyesteramides, polyesters with a fatty chain or alkyds; acrylic and/or vinyl polymers or copolymers; acrylic-silicone copolymers; polyacrylamides; silicone polymers and their mixtures.

9. Composition according to one of the preceding claims, in which the liquid fatty phase is composed of carbon-comprising, hydrocarbon-comprising and/or silicone oils of mineral, animal, plant or synthetic origin, alone or as a mixture.

10. Composition according to one of the preceding claims, in which the liquid fatty phase is chosen from liquid paraffin or liquid petrolatum, mink oil, turtle oil, soybean oil, perhydrosqualene, sweet almond oil, calophyllum oil, palm oil, parleam oil, grape seed oil, sesame oil, maize oil, rapeseed oil, sunflower oil, cottonseed oil, apricot oil, castor oil, avocado oil, jojoba oil, olive oil or cereal germ oil; esters of lanolic acid, of oleic acid, of lauric acid or of stearic acid; fatty esters, such as isopropyl myristate, isopropyl palmitate, butyl stearate, hexyl laurate, diisopropyl adipate, isononyl isononate, 2-ethylhexyl palmitate, 2-hexyldecyl laurate, 2-octyldecyl palmitate, 2-octyldodecyl myristate or lactate, di (2-ethylhexyl) succinate, diisostearyl malate, glyceryl triisostearate or diglyceryl triisostearate; higher fatty acids, such as myristic acid, palmitic acid, stearic acid, behenic acid, oleic acid, linoleic acid, linolenic acid or isostearic acid; higher fatty alcohols, such as cetanol, stearyl alcohol or oleyl alcohol, linoleyl or linolenyl alcohol, isostearyl alcohol or octyldodecanol; silicone oils, such as PDMS, which are optionally phenylated, such as phenyltrimethicones, or which are optionally substituted by aliphatic and/or aromatic groups or by functional groups, such as hydroxyl, thiol and/or amine groups; polysiloxanes modified by fatty acids, fatty alcohols or polyoxyalkylenes; or volatile oils, such as octamethylcyclotetrasiloxane, decamethylcyclopentasiloxane, hexadecamethylcyclohexasiloxane, heptamethylhexyltrisiloxane, heptamethyloctyltrisiloxane or $C_8$-$C_{16}$ isoparaffins, in particular isododecane.

11. Composition according to one of the preceding claims, in which the liquid fatty phase is chosen from the group comprising:

- non-aqueous liquid compounds having an overall solubility parameter according to the Hansen solubility space of less than 17 $(MPa)^{1/2}$,
- or monoalcohols having an overall solubility parameter according to the Hansen solubility space of less than or equal to 20 $(MPa)^{1/2}$,
- or their mixtures,

12. Composition according to one of Claims 1, 2 and 4 to 11, characterized in that the fatty phase comprises at least one oil which is volatile at room temperature.

13. Composition according to one of the preceding claims, in which the liquid fatty phase comprises at least one non-volatile oil.

14. Composition according to one of Claims 6 to 13, in which the stabilizing agent is chosen from sequential polymers, grafted polymers, random polymers and their mixtures.

15. Composition according to Claim 14, in which the stabilizing agent is chosen from silicone polymers grafted with a hydrocarbon-comprising chain; hydrocarbon-comprising polymers grafted with a silicone chain; grafted copolymers having an insoluble backbone of polyacrylic type with soluble grafts of polyhydroxystearic acid type; grafted or sequential block copolymers comprising at least one block of polyorganosiloxane type and at least one block of a radical polymer; grafted or sequential block copolymers comprising at least one block of polyorganosiloxane type and at least one block of a polyether; copolymers of acrylates or methacrylates of $C_1$-$C_4$ alcohols and of acrylates or methacrylates of $C_8$-$C_{30}$ alcohols; grafted or sequential block copolymers comprising at least one block resulting from the polymerization of a diene and at least one block of a vinyl polymer; grafted or sequential block copolymers comprising at least one block resulting from the polymerization of dienes and at least one block of an acrylic polymer; or grafted or sequential block copolymers comprising at least one block resulting from the polymerization of dienes and at least one block of a polyether.

16. Composition according to Claim 14 or 15, characterized in that the stabilizing agent is a grafted or sequential block copolymer comprising at least one block resulting from the polymerization of a diene and at least one block of a vinyl polymer.

17. Composition according to one of the preceding claims, additionally comprising at least one additional fatty phase chosen from waxes, gums and/or pasty fatty substances of plant, animal, mineral, synthetic or silicone origin, and their mixtures.

18. Composition according to one of Claims 1 and 5 to 16, characterized in that the pulverulent compound is chosen from fillers, pigments, pearlescent agents and their mixtures.

19. Composition according to one of Claims 1 to 18, characterized in that the pulverulent compound represents up to 96.8% of the total weight of the composition.

20. , Composition according to one of Claims 13 to 19, characterized in that the non-volatile oil represents from 0.1 to 10% of the total weight of the composition.

21. Composition according to one of the preceding claims, characterized in that the polymer represents, as dry matter, up to 20% of the total weight of the composition.

22. Composition according to one of the preceding claims, characterized in that the polymer represents, as dry matter, from 2 to 10% of the total weight of the composition.

23. Composition according to one of the preceding claims, characterized in that the liquid fatty phase comprises at least one oil chosen from $C_8$-$C_{16}$ isoparaffins and linear or cyclic silicones having from 2 to 7 silicon atoms, these silicones optionally comprising alkyl groups having from 1 to 10 carbon atoms, and their mixtures.

24. Composition according to one of the preceding claims, which is provided in the form of a compacted, pressed or free powder.

25. Composition according to one of the preceding claims, which is provided in anhydrous form.

26. Composition according to one of the preceding claims, which is provided in the form of a product for caring for and/or for making up the skin and/or lips.

27. Composition according to one of the preceding claims, which is provided in the form of a blusher, eyeshadow, lip product, powder for caring for or making up the face or body, or deodorant powder.

28. Use, in a cosmetic, dermatological, pharmaceutical or hygiene pulverulent composition, of a liquid fatty phase comprising at least 2% by weight, with respect to the total weight of the composition, of polymer which is dispersible in the said liquid fatty phase, as binder and agent for decreasing, indeed eliminating, the transfer of the composition deposited on the lips and/or skin of human beings to a substrate brought in contact with the deposit.

29. Use, in a cosmetic, dermatological, pharmaceutical or hygiene pulverulent composition, of a liquid fatty phase comprising at least 2% by weight, with respect to the total weight of the composition, of polymer which is dispersible in the said liquid fatty phase, as binder and agent for decreasing, indeed eliminating, the migration of the composition deposited on the lips and/or skin of human beings into the folds, wrinkles and fine lines of the skin.

30. Use, in a cosmetic, dermatological, pharmaceutical or hygiene pulverulent composition, of a liquid fatty phase comprising at least 2% by weight, with respect to the total weight of the composition, of polymer which is dispersible in the said liquid fatty phase, as binder and agent for enhancing the hold.

31. Use, in a pulverulent composition comprising at least one pulverulent compound, the particles of which are bonded to one another by a binder, of a volatile liquid fatty phase and of at least 2% by weight, with respect to the total weight of the composition, of polymer which is dispersible in the said liquid fatty phase, as agent for decreasing, indeed eliminating, the transfer of the composition deposited on the skin and/or lips.

32. Use, in a pulverulent composition comprising at least one pulverulent compound, the particles of which are bonded to one another by a binder, of a volatile liquid fatty phase and of at least 2% by weight, with respect to the total weight of the composition, of polymer which is dispersible in the said liquid fatty phase, as agent for decreasing, indeed eliminating, the migration of the composition deposited on the skin and/or lips.

33. Use, in a pulverulent composition comprising at least one pulverulent compound, the particles of which are bonded to one another by a binder, of a volatile liquid fatty phase and of at least 2% by weight, with respect to the total weight of the composition, of polymer which is dispersible in the said liquid fatty phase, as agent for enhancing the hold of the composition deposited on the skin and/or lips.

34. Use according to any one of Claims 28 to 33, characterized in that the polymer can form a film.

35. Process for the cosmetic care of or for making up the lips or skin, which consists in applying, to the lips or skin respectively, a cosmetic composition as defined in Claims 1 to 27,

36. Cosmetic process for limiting, indeed eliminating, the transfer of a composition for making up or caring for the skin or lips onto a substrate other than the skin or lips and/or limiting, indeed eliminating, the migration and/or enhancing the hold of the said composition, the composition comprising at least one pulverulent compound, the particles of which are bonded to one another by a binder, which consists in introducing, into the composition, a liquid fatty phase comprising at least 2% by weight, with respect to the total weight of the composition, of a polymer which is dispersible in the said liquid fatty phase.

**Patentansprüche**

1. Zusammensetzung zur topischen Anwendung in Pulverform, die mindestens eine pulverförmige Verbindung enthält, deren Partikel untereinander über ein Bindemittel verbunden sind, dadurch gekennzeichnet, daß das Bindemittel eine flüssige Fettphase und mindestens 2 Gew.-% eines in der flüssigen Fettphase dispergierbaren Polymers, bezogen auf das Gesamtgewicht der Zusammensetzung, enthält.

2. Zusammensetzung nach Anspruch 1, dadurch gekennzeichnet, daß es sich bei dem Polymer um ein filmbildendes Polymer handelt.

3. Zusammensetzung in Pulverform, die mindestens eine pulverförmige Verbindung enthält, deren Partikel untereinander über ein Bindemittel verbunden sind, dadurch gekennzeichnet, daß das Bindemittel eine kosmetische, dermatologische, hygienische oder pharmazeutische, flüchtige, flüssige Fettphase und mindestens 2 Gew.-% eines in der flüssigen Fettphase dispergierbaren filmbildenden Polymers, bezogen auf das Gesamtgewicht der Zusammensetzung, enthält.

4. Zusammensetzung nach einem der Ansprüche 1 bis 3, die ferner mindestens einen Wirkstoff enthält, der unter den kosmetischen, dermatologischen, hygienischen oder pharmazeutischen Wirkstoffen ausgewählt ist.

5. Zusammensetzung nach einem der Ansprüche 2 bis 4, die ferner mindestens ein Färbemittel enthält.

6. Zusammensetzung nach einem der vorhergehenden Ansprüche, wobei das Polymer in Form von Partikeln vorliegt, die durch mindestens ein Stabilisierungsmittel an der Oberfläche stabilisiert und dispergiert sind.

7. Zusammensetzung nach einem der vorhergehenden Ansprüche, wobei das Polymer unter den durch radikalische Polymerisation hergestellten Polymeren, Polykondensaten, Polymeren natürlicher Herkunft und deren Gemischen ausgewählt ist.

8. Zusammensetzung nach einem der vorhergehenden Ansprüche, wobei das Polymer unter den Polyurethanen, Polyurethan-Acrylpolymeren, Polyharnstoffen, Polyharnstoff/Polyurethanen, Polyester-Polyurethanen, Polyether-Polyurethanen, Polyestern, Polyesteramiden, Polyestern mit Fettkette und Alkydharzen; Acryl- und/oder Vinylpolymeren oder Acryl- und/oder Vinylcopolymeren; Acryl-Silicon-Copolymeren; Polyacrylamid; Siliconpolymeren und deren Gemischen ausgewählt ist.

9. Zusammensetzung nach einem der vorhergehenden Ansprüche, dadurch gekennzeichnet, daß die flüssige Fettphase aus Ölen mineralischen, tierischen, pflanzlichen oder synthetischen Ursprungs, Ölen auf Kohlenstoffbasis, Kohlenwasserstoffölen und/oder Siliconölen oder Gemischen dieser Öle besteht.

10. Zusammensetzung nach einem der vorhergehenden Ansprüche, wobei die flüssige Fettphase unter Paraffinöl, Vaselineöl, Nerzöl, Schildkrötenöl, Sojaöl, Perhydrosqualen, Süßmandelöl, Calophyllumöl, Palmöl, Parleam, Traubenkernöl, Sesamöl, Maisöl, Rapsöl, Sonnenblumenöl, Baumwollöl, Aprikosenöl, Ricinusöl, Avocadoöl, Jojobaöl, Olivenöl oder Öl von Getreidekeimen; Estern von Lanolinsäure, Ölsäure, Laurinsäure und Stearinsäure; Fettsäureestern, wie Isopropylmyristat, Isopropylpalmitat, Butylstearat, Hexyllaurat, Diisopropyladipat, Isononylisononat, 2-Ethylhexylpalmitat, 2-Hexyldecyllaurat, 2-Octyldecylpalmitat, 2-Octyldodecylmyristat, 2-Octyldodecyllactat, 2-Diethylhexylsuccinat, Diisostearylmalat, Glycerintriisostearat oder Diglycerintriisostearat; höheren Fettsäuren, wie Myristinsäure, Palmitinsäure, Stearinsäure, Behensäure, Ölsäure, Linolsäure, Linolensäure oder Isostearinsäure; höheren Fettalkoholen, wie Cetanol, Stearylalkohol, Oleylalkohol, Linoleylalkohol, Linolenylalkohol, Isostearylalkohol oder Octyldodecanol; Siliconölen, wie PDMS, die gegebenenfalls Phenylgruppen enthalten, beispielsweise Phenyltrimethicone, oder gegebenenfalls mit aliphatischen und/oder aromatischen Gruppen oder funktionellen Gruppen substituiert sind, wie Hydroxy-, Thiol- und/oder Aminogruppen; mit Fettsäuren, Fettalkoholen oder Polyoxyalkylenen modifizierten Polysiloxanen; flüchtigen Ölen, wie Octamethylcyclotetrasiloxan, Decamethylcyclopentasiloxan, Hexamethylcyclohexasiloxan, Heptamethylhexyltrisiloxan, Heptamethyloctyltrisiloxan oder $C_{8-16}$-Isoparaffinen und insbesondere Isododecan ausgewählt ist.

11. Zusammensetzung nach einem der vorhergehenden Ansprüche, wobei die flüssige Fettphase ausgewählt ist unter:

    - nicht wäßrigen, flüssigen Verbindungen mit einem Gesamtsolubilitätsparameter nach dem Solubilitätsraum von HANSEN unter 17 $(MPa)^{1/2}$,
    - Monoalkoholen mit einem Gesamtsolubilitätsparameter nach dem Solubilitätsraum von HANSEN von höchstens 20 $(MPa)^{1/2}$, oder
    - deren Gemischen.

12. Zusammensetzung nach einem der Ansprüche 1, 2 und 4 bis 11, dadurch gekennzeichnet, daß die Fettphase mindestens ein bei Raumtemperatur flüchtiges Öl enthält.

13. Zusammensetzung nach einem der vorhergehenden Ansprüche, wobei die flüssige Fettphase min-

**15**

destens ein nicht flüchtiges Öl enthält.

14. Zusammensetzung nach einem der Ansprüche 6 bis 13, wobei das Stabilisierungsmittel unter den Blockpolymeren, Pfropfpolymeren, statistischen Polymeren und deren Gemischen ausgewählt ist.

15. Zusammensetzung nach Anspruch 14, wobei das Stabilisierungsmittel ausgewählt ist unter: den mit einer Kohlenwasserstoffkette gepfropften Siliconpolymeren; den mit einer Siliconkette gepfropften Kohlenwasserstoffpolymeren; den gepfropften Copolymeren, die ein unlösliches Grundgerüst vom Polyacryltyp mit löslichen Pfropfzweigen vom Polyhydroxystearinsäuretyp aufweisen; den gepfropften oder sequentiellen Blockcopolymeren, die mindestens einen Block vom Polyorganosiloxantyp und mindestens einen Block eines durch radikalische Polymerisation hergestellten Polymers enthalten; den gepfropften oder sequentiellen Blockcopolymeren, die mindestens einen Block vom Polyorganosiloxantyp und mindestens einen Polyetherblock aufweisen; den Copolymeren von Acrylaten oder Methacrylaten von $C_{1-4}$-Alkoholen und Acrylaten oder Methacrylaten von $C_{8-30}$-Alkoholen; den gepfropften oder sequentiellen Blockcopolymeren, die mindestens einen Block, der bei der Polymerisation eines Diens gebildet wird, und mindestens einen Vinylpolymerblock enthalten; den gepfropften oder sequentiellen Blockcopolymeren, die mindestens einen Block, der bei der Polymerisation eines Diens gebildet wird, und mindestens einen Acrylpolymerblock enthalten; und den gepfropften oder sequentiellen Blockcopolymeren, die mindestens einen Block, der bei der Polymerisation eines Diens gebildet wird, und mindestens einen Polyetherblock enthalten.

16. Zusammensetzung nach Anspruch 14 oder 15, dadurch gekennzeichnet, daß das Stabilisierungsmittel ein gepfropftes oder sequentielles Blockcopolymer ist, das mindestens einen Block, der bei der Polymerisation eines Diens gebildet wird, und mindestens einen Vinylpolymerblock enthält.

17. Zusammensetzung nach einem der vorhergehenden Ansprüche, die ferner mindestens eine zusätzliche Fettphase enthält, die unter den Wachsen, Gummen(Gummis) und/oder pastösen Fettsubstanzen pflanzlichen, tierischen, mineralischen oder synthetischen Ursprungs oder auf Siliconbasis und deren Gemischen ausgewählt ist.

18. Zusammensetzung nach einem der Ansprüche 1, 5 bis 16, dadurch gekennzeichnet, daß die pulverförmige Verbindung unter den Füllstoffen, Pigmenten und Perlglanzpigmenten und deren Gemischen ausgewählt ist.

19. Zusammensetzung nach einem der Ansprüche 1 bis 18, dadurch gekennzeichnet, daß die pulverförmige Verbindung bis zu 96,8 % des Gesamtgewichts der Zusammensetzung ausmacht.

20. Zusammensetzung nach einem der Ansprüche 13 bis 19, dadurch gekennzeichnet, daß das nicht flüchtige Öl 0,1 bis 10 % des Gesamtgewichts der Zusammensetzung ausmacht.

21. Zusammensetzung nach einem der vorhergehenden Ansprüche, dadurch gekennzeichnet, daß das Polymer (als Trockensubstanz) bis zu 20 % des Gesamtgewichts der Zusammensetzung ausmacht.

22. Zusammensetzung nach einem der vorhergehenden Ansprüche, dadurch gekennzeichnet, daß das Polymer (als Trockensubstanz) 2 bis 10 % des Gesamtgewichts der Zusammensetzung ausmacht.

23. Zusammensetzung nach einem der vorhergehenden Ansprüche, dadurch gekennzeichnet, daß die flüssige Fettphase mindestens ein Öl enthält, das unter den $C_{8-16}$-Isoparaffinen und den linearen oder cyclischen Siliconen mit 2 bis 7 Siliciumatomen, wobei die Silicone gegebenenfalls Alkylgruppen mit 1 bis 10 Kohlenstoffatomen aufweisen, oder deren Gemischen ausgewählt ist.

24. Zusammensetzung nach einem der vorhergehenden Ansprüche, die in Form eines kompaktierten, gepressten oder freien Pulvers vorliegt.

25. Zusammensetzung nach einem der vorhergehenden Ansprüche, die in wasserfreier Form vorliegt.

26. Zusammensetzung nach einem der vorhergehenden Ansprüche, die in Form eines Produkts zur Pflege und/oder zum Schminken der Haut und/oder der Lippen vorliegt.

27. Zusammensetzung nach einem der vorhergehenden Ansprüche, die in Form von Wangenrouge oder Lidschatten, als dekorativer oder pflegender Lippenstift, als Pulver zur Pflege oder zum Schminken des Gesichts oder des Körpers, als pulverförmiges Deodorant oder als Rouge vorliegt.

28. Verwendung einer flüssigen Fettphase, die mindestens 2 Gew.-% eines in der flüssigen Fettphase dispergierbaren Polymers, bezogen auf das Gesamtgewicht der Zusammensetzung, enthält, in einer kosmetischen, dermatologischen, pharmazeutischen oder hygienischen, pulverförmigen Zusammensetzung als Bindemittel und als Mittel, um das Übertragen der auf den Lippen und/oder der Haut abgeschiedenen Zusammensetzung auf einen Träger, der mit der abgeschiedenen Zusammensetzung in Kontakt kommt, zu vermindern oder sogar zu verhindern.

29. Verwendung einer flüssigen Fettphase, die mindestens 2 Gew.-% eines in der flüssigen Fettphase dispergierbaren Polymers, bezogen auf das Gesamtgewicht der Zusammensetzung, enthält, in einer kosmetischen, dermatologischen, pharmazeutischen oder hygienischen, pulverförmigen Zusammensetzung als Bindemittel und als Mittel, um die Migration der auf den menschlichen Lippen und/oder der Haut und in den Körperfalten, Falten und Fältchen der Haut abgeschiedenen Zusammensetzung zu vermindern oder sogar zu verhindern.

30. Verwendung einer flüssigen Fettphase, die mindestens 2 Gew.-% eines in der flüssigen Fettphase dispergierbaren Polymers, bezogen auf das Gesamtgewicht der Zusammensetzung, enthält, in einer kosmetischen, dermatologischen, pharmazeutischen oder hygienischen, pulverförmigen Zusammensetzung als Bindemittel und als Mittel zur Erhöhung der Haftung.

31. Verwendung einer flüchtigen, flüssigen Fettphase und mindestens 2 Gew.-% eines in der flüssigen Fettphase dispergierbaren Polymers, bezogen auf das Gesamtgewicht der Zusammensetzung, in einer Zusammensetzung in Pulverform, die mindestens eine pulverförmige Verbindung enthält, deren Partikel untereinander über ein Bindemittel verbunden sind, als Mittel, um den Transfer der auf der Haut und/oder den Lippen abgeschiedenen Zusammensetzung zu vermindern oder sogar zu verhindern.

32. Verwendung einer flüchtigen, flüssigen Fettphase und mindestens 2 Gew.-% eines in der flüssigen Fettphase dispergierbaren Polymers, bezogen auf das Gesamtgewicht der Zusammensetzung, in einer Zusammensetzung in Pulverform, die mindestens eine pulverförmige Verbindung enthält, deren Partikel untereinander über ein Bindemittel verbunden sind, als Mittel, um die Migration der auf der Haut und/oder den Lippen abgeschiedenen Zusammensetzung zu vermindern oder sogar zu verhindern.

33. Verwendung einer flüchtigen, flüssigen Fettphase und mindestens 2 Gew.-% eines in der flüssigen Fettphase dispergierbaren Polymers, bezogen auf das Gesamtgewicht der Zusammensetzung, in einer Zusammensetzung in Pulverform, die mindestens eine pulverförmige Verbindung enthält, deren Partikel untereinander über ein Bindemittel verbunden sind, als Mittel, um die Haftung der auf der Haut und/oder den Lippen abgeschiedenen Zusammensetzung zu erhöhen.

34. Verwendung nach einem der Ansprüche 28 bis 33, dadurch gekennzeichnet, daß das Polymer ein filmbildendes Polymer ist.

35. Verfahren zur kosmetischen Pflege oder zum Schminken der Lippen oder der Haut, das darin besteht, auf die Lippen bzw. die Haut eine kosmetische Zusammensetzung nach den Ansprüchen 1 bis 27 aufzubringen.

36. Kosmetisches Verfahren, um das Übertragen einer Zusammensetzung zum Schminken oder zur Pflege der Haut oder der Lippen auf einen Träger, der von der Haut und den Lippen verschieden ist, zu vermindern oder zu verhindern, die Migration zu verhindern und/oder die Haftung der Zusammensetzung zu erhöhen, wobei die Zusammensetzung mindestens eine pulverförmige Verbindung enthält, deren Partikel untereinander über ein Bindemittel verbunden sind, das darin besteht, eine flüssigen Fettphase, die mindestens 2 Gew.-% eines in der flüssigen Fettphase dispergierbaren Polymers, bezogen auf das Gesamtgewicht der Zusammensetzung, enthält, in die Zusammensetzung einzuarbeiten.